# EUROPEAN PATENT SPECIFICATION

(11) **EP 3 658 048 B1**
(45) Date of publication and mention of the grant of the patent: **09.06.2021**
(21) Application number: 18752205.7
(22) Date of filing: 25.07.2018
(51) Int. Cl.: A61B 17/54, A61B 17/00, A61B 90/00

(54) **A HEAD ASSEMBLY OF A DERMAPLANING DEVICE**
KOPFANORDNUNG EINER DERMAPLANING-VORRICHTUNG
ENSEMBLE DE TÊTE D'UN DISPOSITIF DE DERMAPLANING

(30) Priority: 27.07.2017 GB 201712095
(43) Date of publication of application: 03.06.2020
(73) Proprietor: Reckitt Benckiser Health Limited, Slough, Berkshire SL1 3UH (GB)
(72) Inventor: DAWSON, Samuel, Hull Humberside HU8 7DS (GB); HORNE, Jesse John, Warwick Warwickshire CV34 4AB (GB); WOOD, Daniel John, Hull Humberside HU8 7DS (GB); ZHOU, Xianzhi (Oliver), Guangzhou Guangdong (CN)
(74) Representative: Hodgetts, Catherine Dawn
(86) International application number: PCT/GB2018/052088
(87) International publication number: WO 2019/020994

(56) References cited:
- EP-A1- 3 117 736
- US-A- 3 126 629
- US-A- 3 793 726
- US-A- 5 478 346
- US-A1- 2007 255 298

## Description

Dermaplaning devices are used in skincare. These have a blade which is swept across the skin in order to remove soft hair and dead skin cells. The dermaplaning process is generally undertaken by professionals who will scrape an unprotected blade across the skin.

More recently a number of devices have been developed for home use such as that disclosed in US2015/0073438. This device has a blade assembly which is covered with a removable cap in order to protect the blade assembly when not in use.

The present invention is directed to an improvement of such a dermaplaning device which is intended for home use.

EP3117736A discloses a hand-held device for dermaplaning. The device includes a blade with a safety cage forming an assembly removably mounted to a housing.

US3,126,629 discloses a cutting instrument in which the handle of the instrument is secured by molding or casting of a thermoplastic material about a sharp edge blade, causing the thermoplastic material to form a protective sheath enclosing the cutting edge and if desired other portions of the blade.

US20070255298A1 relates to a blade assembly for a surgical scalpel that includes a blade carrier with a blade attached one end thereof and a protective cover covers the blade and blade holder, where the blade carrier has a detent and the protective cover has a latch in releasable engagement with the detent to hold the protective covering in a protecting position covering said blade assembly and said blade.

The present invention provides a head assembly for a dermaplaning device according to claim 1.

By providing a tab which is connectable to a blade cover by one or more frangible portions, the present invention provides a visual indication to a user that a blade is new and has not been tampered with. However, the cap still continues to function as a removable cap to continue to protect the blade assembly given the presence of the means for removable attachment which remains operable after the removal of the tab.

The tab is preferably configured such that the irreversible deformation requires the complete removal of the tab from the blade cover as this provides a best visual indication of first use. It is possible, however, that the frangible portions could be plastically deformed in some other way without fully removing the tab provided that this deformation were readily apparent visually.

It is desirable for the tab to be highly visually distinctive and also relatively easy to remove. As such, the tab preferably projects from the side of the cap opposite to the blade. It preferably extends in a direction perpendicular to the cap and extends for at least 3mm and preferably at least 4mm. It is preferably configured to be removed by rotation about a single axis. Preferably this single axis is parallel to the direction in which the blade extends. Preferably the cap is fitted on the head such that it is not rotatable about an axis parallel to the single axis.

The means for removable attachment of the cap to the head may be a frictional fit which will retain the cap in place even when inverted. It may alternatively or additionally be a snap fit or a more complex latching arrangement.

Preferably the elongate end is offset from a central axis of the head and wherein the tab does not extend beyond the footprint of the head. This allows a relatively large tab to be accommodated without increasing the footprint of the head.

The, or each, frangible portion preferably comprises a notch creating a thin walled region(s) in the cap. Preferably there are two or more notches on each side of the cap.

The cap and head preferably have a complimentary protrusion and recess to provide the latching mechanism. In this situation, the recess is preferably on the head and the protrusion is on the cap.

Preferably the latching mechanism is engagable by the cap being a press fit onto the head so as to cause a snap fit of the latching mechanism. This provides a simple way to fit the cap onto the head prior to the first use of the device when the frangible portions will be deformed or broken and the cap removed to expose the blade.

The head assembly may be permanently attached to a dermaplaning device. Preferably, however, the head assembly has an attachment mechanism for attachment to the body of a dermaplaning device.

An example of a head assembly of a dermaplaning device in accordance with the present invention will now be described with the referencing accompanying drawings, in which:

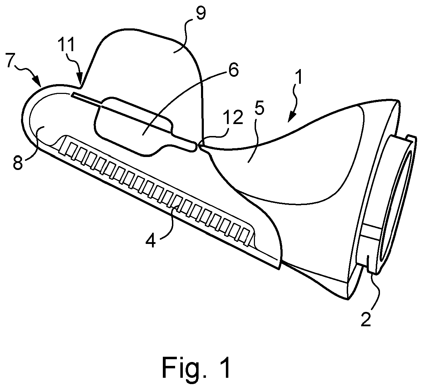

Fig. 1 is a perspective view of the head assembly from a first angle with the cap shown transparent;
Fig. 2 is a side view of the head assembly attached to a body;
Fig. 3 is a perspective view from a second angle of the head assembly attached to a body;
Fig. 4 is a front view of a dermaplaning device including the head;
Fig. 5 is a top view of the device of Fig. 4;
Fig. 6 is a side view of the device of Fig 4;
Fig. 6A is an enlarged drawing of the head of Fig. 6 as designated by the area 6A in Fig 6;
Fig. 7 is a perspective view from above of the device of Fig. 4; and
Fig. 8 is a cross section through the head in an axial plane.

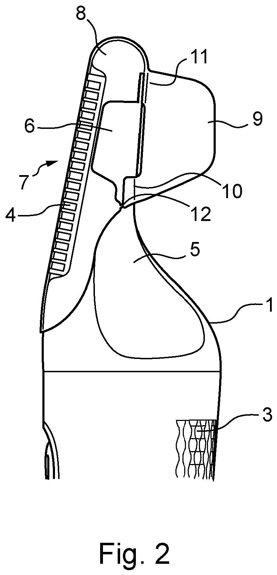

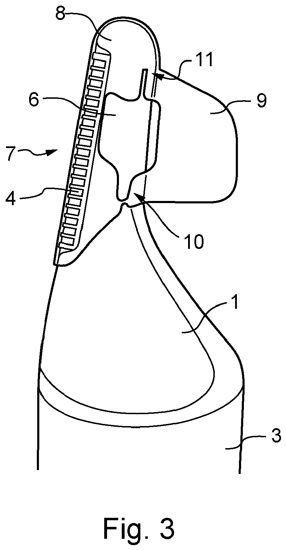

The head 1 comprises a attachment member 2 by virtue of which the head 1 is attached to a body 3 as shown in Fig. 2 and 3. In the simplest example, the blade is a single straight blade 4. Alternatively, however, the blade assembly may comprise two blade portions with toothed edges. One blade portion may be held stationery with the other being vibrated to generate a scissor action between the toothed edges or the two blade positions may be stationery with respect to each other and vibrated as a single unit. The body 3 may then contain a power supply such as a battery, a switch to allow activation of the device and the necessary control circuitry and wiring in order to connect with an electrical connection into the head to cause vibration of the blade assembly in a manner known in the art, for example in the Veet Sensitive Precision™ device.

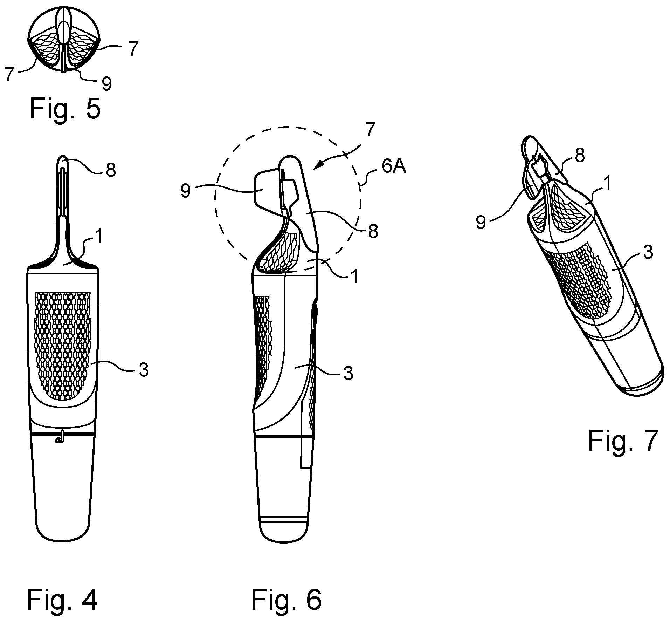

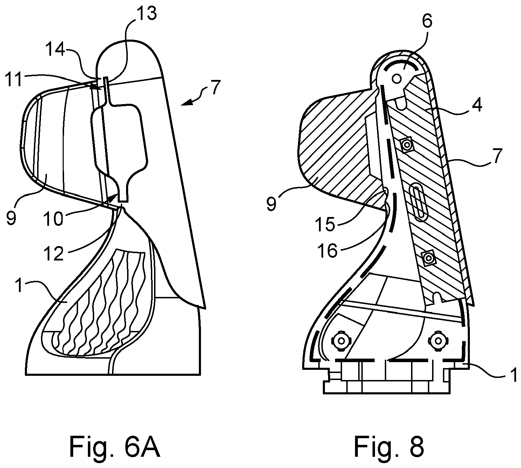

The head 1 comprises a main body 5, with a generally elongate configuration having a pair of recessed gripping surfaces and an axial end portion 6 extending at a slight incline, but generally axially in a direction away from the body 3. The axial end portion 6 is generally relatively flat as can be seen in Figs. 4 and 5. Further, it is offset from the axis of the main body 3 as can be seen in Figs.2, 6 and 6A. The axial end portion 6 is provided with the blade 4 extending along one side of axial end portion 6.

The present invention relates to an adaptation of the cap 7 for such a head 1.

The cap 7 comprises two main portions namely a blade cover 8 and a tab 9. The blade cover 8 forms the main body of the cap and covers a substantial portion of the axial end portion 6 of the head 1 including the blade 4.

The blade cover 8 is designed as an interference fit on the axial end portion 6 such that, in the absence of the tab 9, it will be removably retained on the axial end portion 6. It is also provided with a latching mechanism in the form of, for example, a complimentary protrusion and recess between the blade cover 8 and the head 1 (in the axial end portion 6) to provide a more secure but reversible connection.

Alternatively, the cap 7 may be held on by the sides of the blade cover 8 being resiliently biased towards one another to grip the end axial portion 6.

The tab 9 is connected to the blade cover 8 by lower and upper frangible portions 10, 11.

The lower frangible portion 10 takes the form of a pair of regions of much thinner material 12 one on either side of the tab 9 such that when this thinner material breaks, the tab 9 is free of the blade cover 8. Similarly, the upper frangible portion 11 is formed by a pair of elongate slots 13 as best shown in Fig. 6A which again create thin wall portions 14 on either side of the tab 9. Again, once the material of the thinner portions 14 is sheared the tab 9 is free of the blade cover 8.

As can be seen in the drawings, the tab 9 projects directly away from the blade cover 8 and is at least as wide as the blade cover 8 itself thus presenting a very visually obvious feature. As mentioned above, the axial end portion 6 is offset to a significant extent from the central axis of the body 3. Although the tab 9 extends to a considerable extent, it does not project beyond the footprint of the body 3 such that it does not increase the shelf space required for the device.

The tab 9 has a shoulder 15 (best shown in Fig. 8) which fits into a corresponding recess 16 on the head 1 to prevent removal of the cap 7 from the head 1 when the tab 9 is in place. The cap 7 is sufficiently deformable that it can be press fitted onto the head 1 to allow the complementary shoulder 15, to come into engagement with the recess 16 without breaking the frangible portion 10. However, once the shoulder 15 is located within the recess 16 the cap 7 cannot be removed without destroying at least one of the frangible portions 10, 11.

The user is provided with the head 1 in the form that it is shown in the drawings. It is immediately apparent to them from the highly distinctive tab 9 that the cap 7 has not previously been removed. When the user wants to first use the device, they can readily do so by rotating the large tab 9 about an axis passing through the frangible portions 10, 11 which is generally parallel to the direction of extension of the blade 4. The cap 7 has a non-circular cross section such that it will not rotate as the tab 9 is rotated in order break the frangible portions 10, 11. The removal of the lower frangible portion 10 also removes the shoulder 15 from the recess 16 thereby leaving the interference fit as the only mechanism to retain the cap 7 on the head 1. The tab 9 can then be discarded and the user will subsequently remove and replace the blade cover 8 at will.

## Claims

1. A head assembly for a dermaplaning device, the assembly comprising:
a head (1) having a housing with a elongate end (6) and a blade (4) extending along one side of the elongate end (6);
the assembly further comprising a removable cap (7) which is fitted onto the head (1) so as to cover the blade (4), the cap (7) comprising a blade cover (8) which covers the blade (4) and has a means for removable attachment to the head (1),
**characterised in that** the cap (7) comprises a frangible tab (9) connected to the blade cover (8) by one or more frangible portions (10, 11), the tab (9) comprising a latching mechanism for attachment to the head (1), the latching mechanism being configured such that the cap (7) cannot be removed from the head (1) unless at least one frangible portion (10, 11) is irreversibly deformed, the means for removable attachment to the head (1) being positioned such that it continues to function to retain the blade cover (8) on the head (1) following the irreversible deformation of the frangible portion (10, 11).

2. An assembly according to claim 1, wherein the tab (9) is configured such that the irreversible deformation is the complete removal of the tab (9) from the blade cover (8).

3. An assembly according to claim 1 or 2, wherein the tab (9) projects from the side of the cap (7) opposite to the blade (4).

4. An assembly according to any of claims 1 to 3, wherein the tab (9) extends in a direction perpendicular to the cap (7) and extends for at least 3mm and preferably at least 4mm.

5. An assembly according to any preceding claim, wherein the tab (9) is configured to be removed by rotation about a single axis.

6. An assembly according to claim 5, wherein this single axis is parallel to the direction in which the blade (4) extends.

7. An assembly according to claim 5 or claim 6, wherein the cap (7) is fitted on the head such that it is not rotatable about an axis parallel to the single axis.

8. An assembly according to any preceding claim, wherein the elongate end (6) is offset from a central axis of the head (1) and wherein the tab (9) does not extend beyond a footprint of the head (1).

9. An assembly according to any preceding claim wherein there are frangible portions (10, 11) on opposite sides of the cap (7).

10. An assembly according to any one of the preceding claims, wherein the or each frangible portion (10, 11) comprises a notch (13) creating a thin walled region (12, 14) in the cap (7).

11. An assembly according to claim 10, wherein there are two or more notches (13) on each side of the cap (7).

12. An assembly according to any one of the preceding claims, wherein the cap (7) and head (1) have a complimentary protrusion (15) and recess (16) to provide the latching mechanism.

13. An assembly according to claim 12, wherein the recess is in the head (1) and the protrusion is on the cap (7).

14. An assembly according to any preceding claim, wherein the latching mechanism is engagable by the cap (7) being a press fit onto the head (1) so as to cause a snap fit of the latching mechanism.

15. An assembly according to any preceding claim, wherein the head assembly has an attachment mechanism (2) for attachment to the body (3) of a dermaplaning device.

## Patentansprüche

1. Kopfanordnung für eine Dermaplaning-Vorrichtung, wobei die Anordnung umfasst:
einen Kopf (1), der ein Gehäuse mit einem länglichen Ende (6) und einer Klinge (4) aufweist, die sich entlang einer Seite des länglichen Endes (6) erstreckt;
wobei die Anordnung ferner eine abnehmbare Kappe (7) umfasst, die auf dem Kopf (1) angebracht ist, um die Klinge (4) abzudecken, wobei die Kappe (7) eine Klingenabdeckung (8) umfasst, die die Klinge (4) abdeckt und eine Einrichtung zur entfernbaren Befestigung am Kopf (1) aufweist,
**dadurch gekennzeichnet, dass** die Kappe (7) eine zerbrechliche Lasche (9) umfasst, die mit der Klingenabdeckung (8) durch einen oder mehrere zerbrechliche Abschnitte (10, 11) verbunden ist, wobei die Lasche (9) einen Verriegelungsmechanismus zur Befestigung am Kopf (1) umfasst, wobei der Verriegelungsmechanismus so konfiguriert ist, dass die Kappe (7) nicht vom Kopf (1) entfernt werden kann, es sei denn, mindestens ein zerbrechlicher Abschnitt (10, 11) wird irreversibel verformt, wobei die Einrichtung zur entfernbaren Befestigung am Kopf (1) so positioniert ist, dass sie weiterhin funktioniert, um die Klingenabdeckung (8) nach der irreversiblen Verformung des zerbrechlichen Teils (10, 11) auf dem Kopf (1) zu halten.

2. Anordnung nach Anspruch 1, wobei die Lasche (9) so konfiguriert ist, dass die irreversible Verformung das vollständige Entfernen der Lasche (9) von der Klingenabdeckung (8) ist.

3. Anordnung nach Anspruch 1 oder 2, wobei die Lasche (9) von der der Klinge (4) gegenüberliegenden Seite der Kappe (7) vorsteht.

4. Anordnung nach einem der Ansprüche 1 bis 3, wobei sich die Lasche (9) in einer Richtung senkrecht zur Kappe (7) erstreckt und sich über mindestens 3 mm und vorzugsweise mindestens 4 mm erstreckt.

5. Anordnung nach einem vorhergehenden Anspruch, wobei die Lasche (9) so konfiguriert ist, dass sie durch Drehen um eine einzelne Achse entfernt werden kann.

6. Anordnung nach Anspruch 5, wobei diese einzelne Achse parallel zu der Richtung ist, in der sich die Klinge (4) erstreckt.

7. Anordnung nach Anspruch 5 oder Anspruch 6, wobei die Kappe (7) so auf dem Kopf angebracht ist, dass sie nicht um eine Achse parallel zur einzelnen Achse drehbar ist.

8. Anordnung nach einem vorhergehenden Anspruch, wobei das längliche Ende (6) von einer Mittelachse des Kopfes (1) versetzt ist, und wobei sich die Lasche (9) nicht über eine Grundfläche des Kopfes (1) hinaus erstreckt.

9. Anordnung nach einem vorhergehenden Anspruch, wobei sich auf gegenüberliegenden Seiten der Kappe (7) zerbrechliche Abschnitte (10, 11) befinden.

10. Anordnung nach einem der vorhergehenden Ansprüche, wobei der oder jeder zerbrechliche Abschnitt (10, 11) eine Kerbe (13) umfasst, die einen dünnwandigen Bereich (12, 14) in der Kappe (7) erzeugt.

11. Anordnung nach Anspruch 10, wobei auf jeder Seite der Kappe (7) zwei oder mehr Kerben (13) vorhanden sind.

12. Anordnung nach einem der vorhergehenden Ansprüche, wobei die Kappe (7) und der Kopf (1) einen komplementären Vorsprung (15) und eine Aussparung (16) aufweisen, um den Verriegelungsmechanismus bereitzustellen.

13. Anordnung nach Anspruch 12, wobei sich die Aussparung im Kopf (1) befindet und sich der Vorsprung auf der Kappe (7) befindet.

14. Anordnung nach einem vorhergehenden Anspruch, wobei der Verriegelungsmechanismus durch die Kappe (7) in Eingriff bringbar ist, die eine Presspassung auf dem Kopf (1) ist, um einen Schnappverschluss des Verriegelungsmechanismus zu bewirken.

15. Anordnung nach einem der vorhergehenden Ansprüche, wobei die Kopfanordnung einen Befestigungsmechanismus (2) zur Befestigung an dem Körper (3) einer Dermaplaning-Vorrichtung aufweist.

## Revendications

1. Ensemble de tête pour un dispositif de dermaplaning, l'ensemble comprenant :
une tête (1) ayant un boîtier doté d'une extrémité allongée (6) et une lame (4) s'étendant le long d'un côté de l'extrémité allongée (6) ;
l'ensemble comprenant en outre un capuchon amovible (7) qui est monté sur la tête (1) de manière à recouvrir la lame (4), le capuchon (7) comprenant un couvre-lame (8) qui recouvre la lame (4) et a un moyen de fixation amovible à la tête (1),
**caractérisé en ce que** le capuchon (7) comprend une patte cassable (9) reliée au couvre-lame (8) par une ou plusieurs parties cassables (10, 11), la patte (9) comprenant un mécanisme de verrouillage pour une fixation à la tête (1), le mécanisme de verrouillage étant conçu de sorte que le capuchon (7) ne puisse être retiré de la tête (1) que si au moins une partie cassable (10, 11) est déformée de manière irréversible, le moyen de fixation amovible à la tête (1) étant positionné de sorte qu'il continue à fonctionner pour retenir le couvre-lame (8) sur la tête (1) suite à la déformation irréversible de la partie cassable (10, 11).

2. Ensemble selon la revendication 1, dans lequel la patte (9) est conçue de sorte que la déformation irréversible corresponde au retrait complet de la patte (9) du couvre-lame (8).

3. Ensemble selon la revendication 1 ou 2, dans lequel la patte (9) fait saillie depuis le côté du capuchon (7) opposé à la lame (4).

4. Ensemble selon l'une quelconque des revendications 1 à 3, dans lequel la patte (9) s'étend dans une direction perpendiculaire au capuchon (7) et s'étend d'au moins 3 mm et de préférence d'au moins 4 mm.

5. Ensemble selon l'une quelconque des revendications précédentes, dans lequel la patte (9) est conçue pour être retirée par rotation autour d'un axe unique.

6. Ensemble selon la revendication 5, dans lequel cet axe unique est parallèle à la direction dans laquelle la lame (4) s'étend.

7. Ensemble selon la revendication 5 ou la revendication 6, dans lequel le capuchon (7) est monté sur la tête de sorte qu'il ne puisse pas tourner autour d'un axe parallèle à l'axe unique.

8. Ensemble selon l'une quelconque des revendications précédentes, dans lequel l'extrémité allongée (6) est décalée par rapport à un axe central de la tête (1) et dans lequel la patte (9) ne s'étend pas au-delà d'une empreinte de la tête (1).

9. Ensemble selon l'une quelconque des revendications précédentes, dans lequel des parties cassables (10, 11) se trouvent sur des côtés opposés du capuchon (7).

10. Ensemble selon l'une quelconque des revendications précédentes, dans lequel la ou chaque partie cassable (10, 11) comprend une encoche (13) créant une région à paroi mince (12, 14) dans le capuchon (7).

11. Ensemble selon la revendication 10, dans lequel deux encoches (13) ou plus se trouvent de chaque côté du capuchon (7).

12. Ensemble selon l'une quelconque des revendications précédentes, dans lequel le capuchon (7) et la tête (1) ont une saillie (15) et un évidement (16) complémentaires pour former le mécanisme de verrouillage.

13. Ensemble selon la revendication 12, dans lequel l'évidement est dans la tête (1) et la saillie est sur le capuchon (7).

14. Ensemble selon l'une quelconque des revendications précédentes, dans lequel le mécanisme de verrouillage peut être mis en prise par le capuchon (7), constituant un ajustement serré sur la tête (1), de manière à provoquer un ajustement par encliquetage du mécanisme de verrouillage.

15. Ensemble selon l'une quelconque des revendications précédentes, dans lequel l'ensemble de tête a un mécanisme de fixation (2) pour la fixation au corps (3) d'un dispositif de dermaplaning.
